Europäisches Patentamt

European Patent Office

Office européen des brevets

(11) Publication number: **0 166 476**
**B1**

# EUROPEAN PATENT SPECIFICATION

(45) Date of publication of patent specification: **03.05.89**

(21) Application number: **85200830.9**

(22) Date of filing: **23.05.85**

(51) Int. Cl.⁴: **A 61 M 15/00** // A61M11/06

(54) Aerosol generator-inhalator.

(30) Priority: **28.05.84 NL 8401699**

(43) Date of publication of application:
**02.01.86 Bulletin 86/01**

(45) Publication of the grant of the patent:
**03.05.89 Bulletin 89/18**

(84) Designated Contracting States:
**AT BE CH DE FR GB IT LI NL SE**

(56) References cited:
**WO-A-80/01645**
**WO-A-83/03976**
**DE-A-2 722 701**
**DE-A-3 025 936**
**FR-A- 498 215**
**FR-A-1 036 384**
**FR-A-2 136 920**
**FR-A-2 440 742**
**FR-E- 86 888**
**NL-C- 63 806**
**US-A-2 201 701**
**US-A-2 535 844**
**US-A-2 868 198**
**US-A-4 268 460**

(73) Proprietor: MALLINCKRODT, INC.(a Missouri
corporation)
675 McDonnell Boulevard P.O. Box 5840
St. Louis Missouri 63134 (US)

(72) Inventor: Plomp, Adrianus
c/o OCTROOIBUREAU ZOAN B.V. Apollolaan
151
NL-1077 AR Amsterdam (NL)
Inventor: de Jong, Rudolf Barend Jan
c/o OCTROOIBUREAU ZOAN B.V. Apollolaan
151
NL-1077 AR Amsterdam (NL)

(74) Representative: Swaters, Pieter D. et al
OCTROOIBUREAU ZOAN B.V. P.O. Box 140
NL-1380 AC Weesp (NL)

(56) References cited:
ANN. OCCUP. HYG., vol. 19,1976, pages 363-
366; Pergamon Press, GB. P. KOTRAPPA et al.:
"The performance of nebuliser and air ejector
made from sections of hypodermic needles"

Courier Press, Leamington Spa, England.

## Description

The invention relates to an aerosol generator-inhalator adapted to bring liquid pharmaceutical and diagnostic compositions into a condition suitable for inhalation. Such a device, also referred to as an atomiser, nebuliser or inhalator in the technical literature, serves to convert a liquid composition into an aerosol form which is then inhaled by a patient. When the liquid composition is a radioactive diagnostic, the resulting radioactive aerosol, as described in United States Patent Specification 4,094,317, may be used, after inhaling by the patient, for lung scintigraphy or lung ventilation studies. The device described in the said Patent Specification comprises a conventional ultrasonic aerosol generator.

The invention relates more in particular to an aerosol generator-inhalator of the kind mentioned in the opening paragraph, comprising in a mutually sealing connection a nebulizer and means suitable for inhaling the aerosol.

Every known aerosol generator has for its disadvantage that during use the yield of inhalable aerosol is small with respect to the quantity of composition presented for atomising. The actual nebulizer produces an aerosol having a considerable spreading in particle size. The larger particles will usually not reach the bronchial tubes of the patient but on the way will deposit on the wall of the generator-inhalator system.

If they do reach the bronchial tubes of the patient, they will not be able, due to their large diameter, to penetrate deeply into the lungs, in particular in the air saccules of the lung or alveoli, but will remain stuck in the central bronchial tubes. However, for therapeutic, but in particular for diagnostic purposes, it is often highly desirable for the liquid composition brought in an aerosol form to penetrate into the lungs as deeply as possible, hence into the alveoli. In order to avoid that much of the composition is present in the form of too large particles a baffle or a wall serving as a baffle is placed in the nebulizer at a short distance in front of the nebulizing means. As a result of this the larger particles are withheld and are returned to the liquid to be atomised through a discharge gutter or in a different way. Although the above-mentioned disadvantage is obviated by this, another serious disadvantage is introduced, namely that a comparatively large part of the often expensive composition to be atomised remains adsorbed on the baffle or the wall serving as a baffle, and on the discharge system connected thereto, and will hence not be available anymore for atomising. The more expensive the medicament or diagnostic, the more serious will be this disadvantage: in this connection may be considered modern medicaments which can be administered through the mucous membranes of the lungs, but also, and in particular, expensive, short-living radioisotopes for lung examination. A promising development in the field of short-living radioisotopes is gold-195$m$ which recently can be produced by means of a suitable generator from mercury-195$m$ and is now available on a commercial scale for diagnostic purposes (see Netherlands Patent Application 8002235). Such short-living radioisotopes such as gold-195$m$ having a half-life of only approximately 30 seconds, are certainly not suitable to be atomised by means of a nebulizer comprising a baffle. The yield per unit of time of aerosol suitable for inhalation would be too small in such a device.

An aerosol generator having an evaporation chamber is known from Swedish Patent Application 81-556. In this evaporation chamber the liquid evaporates from the atomised droplets by heating. After leaving the evaporation chamber, a dry aerosol in an oversaturated vapour is formed. The dry aerosol particles then may serve as condensation nuclei for the vapour, so that the aerosol particles will regain their original size, so will have an equally large diameter as immediately after atomisation. As a consequence the risk of deposition on the walls of the generator system is great and the particles deposit in the respiratory system more quickly. Therefore such a device is not suitable for the object of the present invention.

An aerosol generator-inhalator is further known from DE-A-3025936. Said known apparatus comprises an electrically heatable reservoir connected with a spraying unit, to which a treatment assembly having a guiding channel and a mouthpiece can be connected. This apparatus is intended to solve two problems, viz. (a) to allow both mouth and nose-inhalation with a single treatment assembly, and (b) to reduce the temperature of the vapour jet from approx. 98°C to a temperature acceptable for inhalation with the aid of the treatment assembly. For these purposes the guiding channel of the treatment assembly is provided with air-inlet apertures and the mouthpiece is produced in a special form.

It is the object of the present invention to provide an aerosol generator-inhalator which does not exhibit the disadvantages mentioned above, viz. (1) low yield of inhalable aerosol with respect to the quantity of composition presented for nebulizing, (2) adsorption of a relatively large portion of the composition to be nebulized on a baffle or a wall serving as a baffle and in the discharge system connected thereto, and (3) a premature deposition of the particles due to an oversaturated vapour environment after leaving a heated section, e.g. by using a heatable evaporation chamber.

This object can be achieved by an aerosol generator-inhalator adapted to bring liquid pharmaceutical and diagnostic compositions into a condition suitable for inhalation, comprising a nebulizer, means suitable for the inhalation of the aerosol, a chamber positioned between nebulizer and inhalation means, whereby the nebulizer comprises at least one supply member communicating with at least one liquid reservoir, the open end of the supply member or members being positioned close to the wing nozzle of an air supply, said wing nozzle facing said chamber; which aerosol generator-inhalator is characterized, according to the present invention,

(i) in that said chamber is an aerosol-drying chamber which comprises a member for supplying drying air and which sealingly connects the nebulizer to the inhalation means; (ii) in that said supply member is hypodermic needle shaped; (iii) in that a baffle or a wall serving as a baffle towards the aerosol-drying chamber is missing in the nebulizer, so that the atomized liquid can spray directly into the aerosol-drying chamber when the generator is used; and (iv) in that said air supply is connected to a compressed air supply means, suitable to cause air to flow out of the wing nozzle at a pressure of 200 to 600 kPa in a quantity of 40 to 80 liters per minute per $mm^2$ of surface area of the wing nozzle of the air supply, so that the liquid composition is atomized completely or substantially completely when the generator is used.

Although the aerosol generator-inhalator according to the invention is particularly suitable for bringing very short-living radioisotopes, such as gold-195m, into a condition suitable for inhalation, the device is certainly not restricted thereto. As a matter of fact it has been found that the use of an aerosol-drying chamber presents the great advantage that the medicament or diagnostic brought in an aerosol form, on its way does not deposit or deposits hardly on the wall of the generator-inhalator system, but substantially entirely remains available for inhalation.

A special feature of the invention is the possibility to employ a non aqueous carrier fluid as carrier substance for the pharmaceutically active substance to be nebulized. In general a living organism will not tolerate inhalation of aerosols of such non-aqueous fluids because these aerosols have a strongly irritant action on the respiratory system. The nature of certain pharmaceuticals may require volatile organic solvents as carrier fluid. An aerosol acceptable to the individual who inhales it will only be possible in case the concentration of the vapour of organic solvent in the inhaled air is kept low. The design of the aerosol generator-inhalator of the present invention makes such possible because very small amounts of fluid may be nebulized with virtually 100% production of aerosol. The aerosol drying chamber will allow the droplets of organic solvent to evaporate immediately thus giving raise to a dry aerosol of solid particles of very small dimensions. The drying process of an aerosol involving an organic solvent will in general proceed so fast that within short distance from the nebulizer the solid dry particles already emerge, resulting in an aerosol which has very little tendency to adhere to internal surfaces of the generator-inhalator, with as consequence that a very high percentage of the nebulized pharmaceutical becomes available for inhalation.

In a suitable embodiment said nebulizer is constructed in such manner, that the open end of the liquid supply member or members is positioned at a short distance before the wing nozzle of the hypodermic needle-shaped air supply, which is positioned at an angle of approximately 90 degrees relative to the liquid supply member or members. The distance between the open end of the liquid supply member or members and the wing nozzle of the air supply can be varied at will to reach optimum results in atomizing liquids having different physical properties, e.g. viscosities.

Kotrappa c.s. described a nebulizer having a comparable construction, namely a liquid supply needle and an air supply needle placed at right angles thereto, in Ann. occup. Hyp. Vol. 19, 1976, pp. 363-366. This nebulizer also, however, comprises a baffle which may lead to considerable losses of expensive composition.

In another suitable embodiment said nebulizer is constructed in such manner, that the wing nozzle of the air supply is positioned around the open end of the hypodermic needle-shaped liquid supply member, so that the air is supplied to the liquid to be nebulized through a circumferential gap having an adjustable or fixed width. It has been shown, that this latter embodiment gives adequate results as to fast and complete atomization of the supplied liquid. If the circumferential gap has an adjustable width, this width can be attuned to the physical properties, e.g. the viscosity, of the liquid to be atomized.

In order to reach an atomisation of the liquid composition as complete as possible, the nebulizer of the aerosol generator-inhalator according to the invention is constructed so that the air supply is connected to a compressed air supply means, suitable to cause air to flow out of the wing nozzle at a pressure of 200 to 600 kPa in a quantity of 40 to 80 litres per minute per $mm^2$ of surface area of the wing nozzle of the air supply. When using the generator the liquid composition presented in any arbitrarily selected quantity, up to very small quantities, can be atomised completely or substantially completely in a comparatively short period of time. If the generator is intended to atomise the above-mentioned gold-195m, it may be desirable to atomise approximately 0.5 ml of the gold-195m-containing liquid, i.e. 0.5 ml of eluate which can be obtained from an Hg/Au generator, in approximately 0.5 minute as completely as possible. This has proved possible indeed by means of the above-described aerosol generator according to the invention. Then the size of the generated liquid droplets is at most approximately 10 micrometers.

The volume of the aerosol-drying chamber should be sufficient so that the evaporation in the aerosol-drying chamber to which the liquid particle leaving the nebulizer are exposed, does not lead to the vapour saturation point being reached at which point condensation may occur. The aerosol-drying chamber comprises drying air, while air withdrawn form the aerosol-drying chamber is replenished with likewise drying air through the nebulizer. An improvement can be achieved by supplying extra air not only through the air inlet but also through a ring of holes around the air inlet; it has been found that the possibility of deposition of the particles on the walls can still be reduced hereby. As a result of the drying air in the aerosol-drying chamber the diameter of the aerosol particles generated is considerably reduced, in the case of the above-mentioned atomised gold-195$m$ eluate with approximately a factor of 3; after drying, the maximum diameter of the particles still is at most approx, 3 micrometers. The particle size distribution of the dried aerosol has a geometrically average diameter of approximately 0.4 micrometer and a geometrical range factor of approximately 1.9. Such a particle size distribution is extremely suitable for penetration deep into the lungs, into the alveoli, upon inhaling, even though the particles on their way through the more centrally situated bronchial tubes, grow again by condensation of liquid. The aerosol-drying chamber should preferably have a volume of at least sixty thousand times the volume of the liquid composition to be atomised; so in the case of a composition of 0.5 ml, the aerosol-drying chamber should preferably has a volume of at least 30 litres; these values are defined for room temperature. The inhaled quantity of air is not the same for every patient.

By supplying the average consumption of air during use of the aerosol generator-inhalator, considered over a longer period of time usually no pressure above or below atmospheric pressure will arise in the aerosol-drying chamber. However, in order to be able to compensate for any differences in quantity of inhaled air, the aerosol-drying chamber is preferably manufactured from a flexible material, preferably a flexible plastic, for example, polyethylene.

The aerosol generator-inhalator according to the invention furthermore comprises an inhalation means having a mouthpiece or face mask through which the patient can inhale the generated aerosol. The inhalation means is preferably constructed so as to comprise a mouthpiece. In a preferred embodiment the inhalation means comprises a room divided into three chambers, the chambers respectively being provided with an inlet tube for the aerosol from the aerosol-drying chamber, a mouthpiece, and an outlet tube for expired and surplus air, which outlet may be connected to a filter, if so desired, each chamber within the housing further communicating with the two other chambers through valves. The pressure at which the valve between the chamber communicating with the aerosol-drying chamber and the chamber communicating with the mouthpiece is opened should be adjusted very accurately. On the one hand it must be possible for an enfeebled patient also to open the valve already upon inhaling, on the other hand the valve may not open automatically at a small pressure above atmospheric pressure in the aerosol-drying chamber. As a particular aspect of the invention it has been found that a spring force from approximately 6 to approximately 10 mbar, against which the valve is opened upon inhaling, is particularly suitable for this purpose. The spring force can be obtained, for example, by means of a coil spring which either in the chamber communicating with the aerosol-drying chamber of in the chamber connected to the mouthpiece, bears on the valve in an upright position and keeps it pressed on its valve seat in the closed condition. Upon inhaling, the coil spring is slightly extended in the former case or is slightly compressed in the latter case, so that the valve becomes detached from the valve seat and is opened to admit aerosol from the aerosol-drying chamber to the mouthpiece. The inhalation means is preferably manufactured for the greater part from a form-retaining material which can readily be cleaned and sterilised. When the aerosol generator-inhalator serves to generate a radioactive aerosol, the expired air must be passed through a filter suitable for that purpose, so as to remove radioactive constituents from the expired air.

The use of the above inhalation means is not restricted to the use in an aerosol generator-inhalator as described above, but also extends to the use in other aerosol generator-inhalators, for example, known aerosol generator-inhalators of the disposable type.

The invention will now be described in greater detail with reference to a preferred embodiment shown in the drawings.

In these drawings:
Figure 1 shows in outline a suitable embodiment of an aerosol generator-inhalator according to the invention;
Figure 2 shows a nebulizer;
Figure 3 shows a different embodiment of a nebulizer;
Figure 4 shows an inhalation means in a cross-sectional view, and
Figure 5 shows in outline the operation of the hypodermic needle-shaped nebulizer of Figure 2.

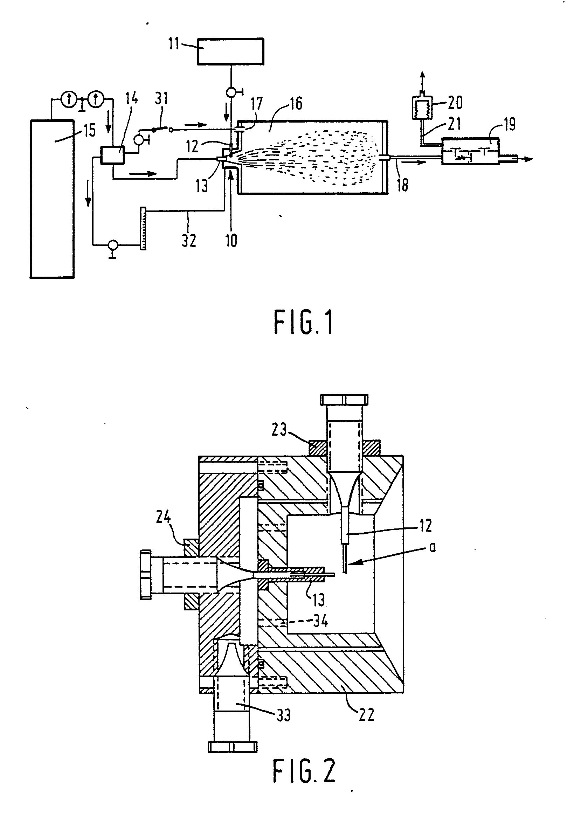

The aerosol generator-inhalator shown in Figure 1 comprises a nebulizer 10 which is composed of an inlet needle 12 connected to a liquid reservoir 11, an air inlet 13 connected, through a distributor 14, to a cylinder 15 with dry compressed air, an aerosol-drying chamber 16 having a volume of approximately 30 litres and comprising an inlet 17 with valve 31 for dry air (herewith the aerosol-drying chamber can be rinsed more quickly), an inlet tube 18 through which the formed aerosol can reach the inhalation means 19, and an outlet tube 21 for expired and surplus air, connected to a filter 20. Extra dry air can be supplied at 32 through a ring of apertures around the air inlet. The aerosol-drying chamber has the form of a cylinder closed at each end (length approximately 60 cm; diameter approximately 25 cm) the end walls of which are formed from circular plates of synthetic material having the necessary perforations, and the side wall consists of flexible polythene sheet. The side wall is connected around the end walls so as to be air-tight.

The nebulizer will be described with reference to Figure 2. In Figure 2, the liquid supply needle and the air supply needle are again referenced 12 and 13, respectively. The needle duct diameter of the liquid supply decreases stepwise to 0.23 mm (at a); the needle is flattened at its end. The dimensions of the air inlet needle are the same as those of the liquid inlet needle. Numeral 33 denotes a nipple for the connection to an inlet for extra air; the air can be admitted to the generator through a ring of apertures 34 around air inlet 13. The needles are firmly connected in a nebulizer housing 22 and can be moved relatively to each other by means of adjusting screws 23 and 24.

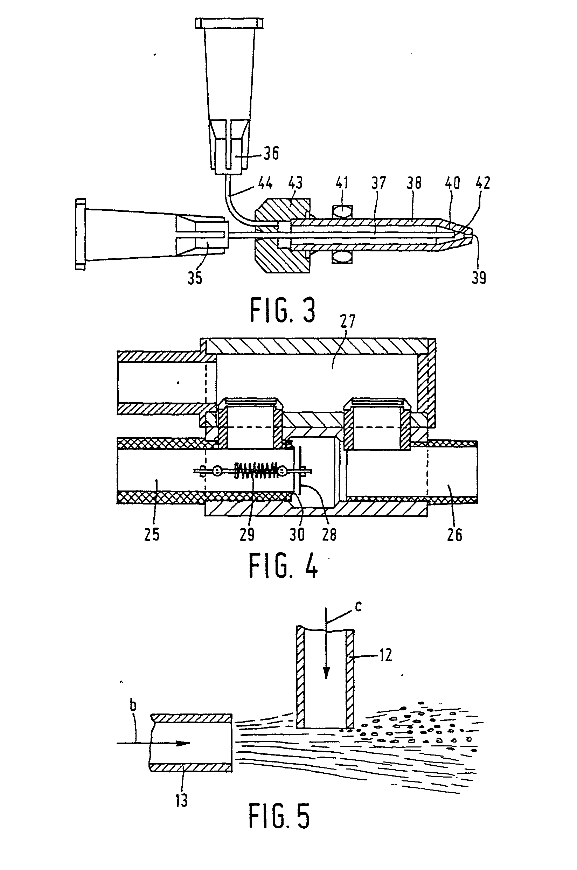

Another suitable embodiment of a nebulizer is schematically shown in cross-sectional view in Figure 3. In Figure 3 the liquid supply and the air supply are provided with reference numerals 35 and 36 respectively. The liquid supply needle 37 extends axially within an outer sleeve 38 nearly to the aperture 39 in the end surface of the inwardly tapering part 40 of said sleeve. The outer sleeve is provided with fastening means 41 on its outer side for attaching the sleeve in a nebulizer housing, not shown in the Figure. A circumferential gap is arranged between the needle tip 42 and the inner wall of the conical part 40 of the sleeve, to allow the supplied air to reach aperture 39. After the width of said gap has been adjusted, needle 37 is sealingly fixed in a holder 43, to which holder the outer sleeve 38 is also firmly and sealingly connected. In a different embodiment needle 37 is not fixed in the holder but is adjustably connected in the boring in the holder, e.g. by means of an adjusting screw, providing a sealed but adjustable connection. The air supply 36 communicates with the interior of the outer sleeve through a hypodermic needle 44, sealingly fixed in a second boring in holder 43. To firmly and sealingly connect the various parts of the nebulizer in and to each other, suitable glues can be used. However, instead of glues other connection means are suitable, such as screw and snap connections.

The inhalation means shown in Figure 4 comprises three chambers 25, 26 and 27 which communicate with each other by means of valves. Chamber 25 communicates with the aerosol-drying chamber (16 in Figure 1), chamber 26 is connected to a mouthpiece not shown in the drawing, and chamber 27 is connected to filter 20 for the expired and surplus air. Valve 28 between chambers 25 and 26 is kept pressed on valve seat 30 by means of a coil spring 29 with a spring force of approximately 8 mbar. At a small pressure below atmospheric pressure in chamber 26, namely from approximately 8 mbar, valve 28 is opened against the action of the spring 29, so that the aerosol can reach the mouthpiece through chamber 25 and 26.

The operation of the nebulizer of Figure 2 can further be explained with reference to Figure 5. Compressed air is supplied through the horizontally arranged injection needle 13 (at b), liquid to be atomised is supplied through the vertical injection needle 12 (at c).

EXAMPLE I

The above-described aerosol generator-inhalator was used to atomise 0.5 ml of gold-195m eluate which comprised approximately 4% sodium thiosulphate in water in addition to a little radioactive gold. The composition was atomised under the influence of dry compressed air which was supplied to the air inlet needle under a pressure of 400 kPa in a quantity of 2.75 litres per minute; the extra supplied drying air was 7.25 litres per minute so that the overall air supply was 10 litres per minute. After exactly 0.5 minute the composition was atomised completely, The distribution of the particle size of the dried aerosol was measured in the mouthpiece of the inhalation means; the geometrically average volume diameter of the particles was 1.5 micrometers.

EXAMPLE II

By means of the above-described generator-inhalator an aerosol was produced from 0.5 ml of a solution containing 50% ethanol and 50% water in which NaCl was dissolved at a concentration of 0.9 grams per liter. The volume of 0.5 ml solution was nebulized in less than 3 minutes and resulted in dry aerosol in which the volume of the generated particles was distributed so that - measured with a laser aerosol sizing instrument - 50% of the volume consisted of particles below 0.9 micrometers diameter.

EXAMPLE III

By means of the above-described generator-inhalator an aerosol was produced from 0.5 ml of a solution containing 100 micrograms of ruthenocene in 1 ml of ethanol 96%. This volume of 0.5 ml was nebulized in less than 3 minutes and did result in a dry particles aerosol with a distribution of the generated particles such that 50% of the volume of the dry particles consisted of particles below 0.3 micrometers diameter.

EXAMPLE IV

An aerosol was generated by means of the above-described generator-inhalator by nebulizing 0.5 ml of a solution containing radioactive Tc99m in a solution equal to the solution described in Example II. It was found that more than 86% of the amount of radioactivity introduced in the generator became available for inhalation at the outlet site of the generator inhalator system.

EXAMPLE V

An aerosol was produced in which latex spheres of 2 micrometers diameter in water containing an addition of acetic acid were nebulized in the above-described generator-inhalator. An inhalable aerosol was obtained in which the particle size was found to be of approx. 2 micrometers diameter.

EXAMPLE VI

An aerosol was produced in which latex spheres of 2 micrometers diameter were suspended in 0.5 ml of ethanol and nebulized in the above-described generator-inhalator. An inhalable aerosol was obtained, that was found to be not irritant to a human subject, in which aerosol the particle size was found to be of approx. 2 micrometers diameter.

EXAMPLE VII

An aerosol was generated by means of the above described aerosol generator-inhalator, wherein the nebulizer of Figure 3 was installed, by nebulizing 0.5 ml of a radioactive complex of technetium-99m and N-[N'-(2,6-dimethylphenyl-)carbamoylmethyl]imidiacetic acid (HIDA) in a solution equal to the solution described in Example II.

Excellent lung images were obtained with this aerosol.

**Claims**

1. An aerosol generator-inhalator adapted to bring liquid pharmaceutical and diagnostic compositions into a condition suitable for inhalation, comprising a nebulizer (10), means (19) suitable for the inhalation of the aerosol, a chamber (16) positioned between nebulizer (10) and inhalation means (19), whereby the nebulizer comprises at least one supply member (12) communicating with at least one liquid reservoir (11), the open end of the supply member or members being positioned close to the wing nozzle of an air supply (13), said wing nozzle facing said chamber; said aerosol generator-inhalator being characterized
(i) in that said chamber (16) is an aerosol-drying chamber which comprises a member (17) for supplying drying air and which sealingly connects the nebulizer (10) to the inhalation means (19); (ii) in that said supply member (12) is hypodermic needle-shaped; (iii) in that a baffle or a wall serving as a baffle towards the aerosol-drying chamber is missing in the nebulizer, so that the atomized liquid can spray directly into the aerosol-drying chamber when the generator is used; and (iv) in that said air supply (13) is connected to a compressed air supply means, suitable to cause air to flow out of the wing nozzle at a pressure of 200 to 600 kPa in a quantity of 40 to 80 liters per minute per mm² of surface area of the wing nozzle of the air supply, so that the liquid composition is atomized completely or substantially completely when the generator is used.

2. An aerosol generator-inhalator as claimed in claim 1, characterized in that the open end of the liquid supply member or members (12) is positioned at a short distance before the wing nozzle of the hypodermic needle-shaped air supply (13), which is positioned at an angle of approximately 90 degrees relative to the liquid supply member or members.

3. An aerosol generator-inhalator as claimed in claim 1, characterized in that the wing nozzle of the air supply (36-44) is positioned around the open end of the hypodermic needle-shaped liquid supply member (35-37), so that the air is supplied to the liquid to be nebulized through a circumferential gap having an adjustable or fixed width.

4. An aerosol generator-inhalator as claimed in any of the preceding claims, characterized in that the aerosol-drying chamber (16) is manufactured from a flexible material, preferably a flexible plastic.

5. An aerosol generator-inhalator as claimed in any of the preceding claims, characterized in that the inhalation means (19) comprises a room divided into three chambers (25, 26, 27), the chambers respectively being provided with an inlet tube for the aerosol from the aerosol-drying chamber, a mouth-piece, and an outlet tube (21) for expired and surplus air, which outlet may be connected to a filter means (20), if so desired, each chamber within the housing further communicating with the two other chambers through valves, and finally upon inhaling the aerosol the valve (28) between the chamber (25) communicating with the aerosol-drying chamber and the chamber (26) connected to the mouthpiece being opened against a spring force of approximately 6 to approximately 10 mbar.

**Patentansprüche**

1. Aerosolgenerator-Inhalator, der geeignet ist, flüssige pharmazeutische und diagnostische Zusammensetzungen in einen für die Inhalation geeigneten Zustand zu bringen, mit einem Zerstäuber (10), einer zum Inhalieren des Aerosols geeigneten Einrichtung (19), einer zwischen dem Zerstäuber (10) und der Inhaliereinrichtung (19) gelegenen Kammer (16), wobei der Zerstäuber zumindest ein Zuführglied (12) aufweist, das mit wenigstens einem Flüssigkeitsbehälter (11) verbunden ist, wobei das offene Ende des Zuführglieds oder der Zuführglieder nahe der Beschleunigungsdüse einer Luftzuführung (13) angeordnet ist, welche Beschleunigungsdüse der Kammer zugewandt ist; welcher Aerosolgenerator-Inhalator dadurch gekennzeichnet ist,
(i) daß die Kammer (16) eine Aerosoltrocknungskammer ist, die ein Glied (17) zur Zuführung von Trocknungsluft aufweist und den Zerstäuber (10) dicht mit der Inhaliereinrichtung (19) verbindet; (ii) daß das Zuführglied (12) Spritzennadel-förmig ist; (iii) daß eine Leitfläche oder eine als Leitfläche dienende Wand zur Aerosoltrocknungskammer hin im Zerstäuber fehlt, so daß die zerstäubte Flüssigkeit direkt in die Aerosoltrocknungskammer sprühen kann, wenn der Generator benützt wird; und (iv) daß die Luftzuführung (13) an eine Druckluftversorgungseinrichtung angeschlossen ist, die geeignet ist, einen Luftstrom aus der Beschleunigungsdüse bei einem Druck von 200 bis 600 kPa in einer Menge von 40 bis 80 1 pro min pro $mm^2$ der Querschnittsfläche der Beschleunigungsdüse der Luftzuführung zu bewirken, so daß die flüssige Zusammensetzung vollständig oder im wesentlichen vollständig zerstäubt wird, wenn der Generator benützt wird.

2. Aerosolgenerator-Inhalator nach Anspruch 1, dadurch gekennzeichnet, daß das offene Ende des Flüssigkeits-Zuführglieds oder der -glieder (12) in einem kleinen Abstand vor der Beschleunigungsdüse der Spritzennadel-förmigen Luftzuführung (13) angeordnet ist, die unter einem Winkel von ungefähr 90° relativ zu dem Flüssigkeits-Zuführglied oder den -gliedern angeordnet ist.

3. Aerosolgenerator-Inhalator nach Anspruch 1, dadurch gekennzeichnet, daß die Beschleunigungsdüse der Luftzuführung (36-44) um das offene Ende des Spritznadel-förmigen Flüssigkeits-Zuführglieds (35-37) herum angeordnet ist, so daß die Luft der zu zerstäubenden Flüssigkeit durch einen Umfangsspalt mit einstellbarer oder fester Breite zugeführt wird.

4. Aerosolgenerator-Inhalator nach einem der vorstehenden Ansprüche, dadurch gekennzeichnet, daß die Aerosoltrocknungskammer (16) aus einem flexiblen Material, vorzugsweise einem flexiblen Kunststoff, hergestellt ist.

5. Aerosolgenerator-Inhalator nach einem der vorstehenden Ansprüche, dadurch gekennzeichnet, daß die Inhaliereinrichtung (19) einen Raum aufweist, der in drei Kammern (25, 26, 27) unterteilt ist, die mit einem Einlaßrohr für das Aerosol aus der Aerosoltrocknungskammer bzw. mit einem Mundstück bzw. mit einem Auslaßrohr (21) für ausgeatmete und überschüssige Luft versehen sind, welcher Auslaß gewünschtenfalls mit einer Filtereinrichtung (20) verbunden sein kann, wobei jede Kammer innerhalb des Gehäuses weiters mit den zwei anderen Kammern über Ventile in Verbindung steht und schließlich bei Inhalieren des Aerosols das Ventil (28) zwischen der mit der Aerosoltrocknungskammer verbundenen Kammer (25) und der mit dem Mundstück verbundenen Kammer (26) entgegen einer Federkraft von ungefähr 6 bis ungefähr 10 mbar öffnet.

## Revendications

1. Générateur-inhalateur d'aérosols adapté pour amener des compositions liquides à usage de diagnostic et pharmaceutique dans un état approprié pour l'inhalation, comprenant un nébuliseur (10), des moyens (19), appropriés pour l'inhalation de l'aérosol, une chambre (16) disposée entre le nébuliseur (10) et les moyens d'inhalation (19), de façon que le nébuliseur comprenne au moins un organe de distribution (12) communiquant avec un réservoir de liquide (11) au moins, l'extrémité ouverte du ou des organes de distribution étant disposée au voisinage de l'embout à ailettes d'une arrivée d'air (13), ledit embout à ailettes faisant face à ladite chambre;

ledit générateur-inhalateur d'aérosols étant caractérisé

(i) en ce que ladite chambre (16) est une chambre de séchage d'aérosol qui comprend un organe (17) de distribution d'air sec et qui relie de façon étanche le nébuliseur (10) aux moyens en forme d'inhalateur (19); (ii) en ce que ledit organe de distribution (12) est en forme d'aiguille hypodermique; (iii) en ce qu'une chicane ou une paroi servant de chicane vers la chambre de séchage de l'aérosol est omise dans le nébuliseur, ce qui fait que le liquide atomisé peut être directement pulvérisé dans la chambre de séchage de l'aérosol quand le générateur est utilisé, et (iv) en ce que ladite distribution d'air (13) est reliée à des moyens de distribution d'air comprimé agencés pour provoquer un écoulement de l'air hors de l'embout à ailettes sous une pression de 200 à 600 kPa, à un débit de 40 à 80 litres par minute, par $mm^2$ de surface d'embout à ailettes de distribution d'air, de façon que la composition liquide est atomisée complètement ou à peu près complètement quand le générateur est utilisé.

2. Générateur-inhalateur d'aérosols selon la revendication 1, caractérisé en ce que l'extrémité ouverte du ou des organes de distribution de liquide (12) est disposée à une courte distance en avant de l'embout à ailettes de la distribution d'air en forme d'aiguille hypodermique (13) qui est positionnée sous un angle de 90° environ par rapport à ou auxdits organes de distribution de liquide.

3. Générateur-inhalateur d'aérosols selon la revendications 1, caractérisé en ce que l'embout à ailettes de la distribution d'air (36-44) est disposée autour de l'extrémité ouverte de l'organe de distribution de liquide en forme d'aiguille hypodermique (35-37), de sorte que l'air est envoyé au liquide à nébuliser par un intervalle périphérique possédant une largeur fixe ou réglable.

4. Générateur-inhalateur d'aérosols selon l'une quelconque des précédentes revendications, caractérisé en ce que la chambre de séchage de l'aérosol (16) est fabriquée à partir d'un matériau souple, de préférence un plastique souple.

5. Générateur-inhalateur d'aérosols selon l'une quelconque des précédentes revendications, caractérisé en ce que les moyens d'inhalation (19) comprennent une enceinte divisée en trois chambres (25, 26, 27), les chambres étant pourvues respectivement d'un tuyau d'entrée d'aérosol venant de la chambre de séchage d'aérosols, d'une embouchure et d'un tuyau de sortie (21) pour l'air expiré et excédentaire, ladite sortie pouvant être raccordée à des moyens en forme de filtre (20) si on le souhalte, chaque chambre dans le boîtier communiquant en outre avec les deux autres chambres par des soupapes et enfin, après inhalation de l'aérosol, la soupape (28) entre la chambre (25) communiquant avec la chambre de séchage d'aérosol et la chambre (26) reliée à l'embouchure étant ouverte contre une force élastique de 6 environ à 10 mbar environ.

FIG.1

FIG.2

FIG. 3

FIG. 4

FIG. 5